# EUROPEAN PATENT SPECIFICATION

(11) **EP 1 319 089 B1**
(45) Date of publication and mention of the grant of the patent: **01.12.2010**
(21) Application number: 01975218.7
(22) Date of filing: 17.09.2001
(51) Int. Cl.: C12Q 1/70, C12Q 1/68, C12Q 1/18, C12Q 1/04, G01N 33/53, A61K 49/00, A61K 39/42, A61K 39/23

(54) **IN VITRO ASSAY FOR MEASURING THE IMMUNOGENICITY OF A VACCINE**
IN VITRO TESTVERFAHREN ZUR BESTIMMUNG DER IMMUNOGENIZITÄT EINES IMPFSTOFFES
DOSAGE IN VITRO DESTINE A MESURER L'ANTIGENICITE D'UN VACCIN

(30) Priority: 18.09.2000 US 233439 P
(43) Date of publication of application: 18.06.2003
(73) Proprietor: MedImmune, LLC, Gaithersburg, MD 20878 (US)
(72) Inventor: SCHENERMAN, Mark, A., Reisterstown, MD 21136 (US); WANG, Sheau-Chiann, Gaithersburg, MD 20878 (US); STROUSE, Robert, J., Derwood, MD 20855 (US); SUZICH, JoAnn, Washington Grove, MD 20882 (US); WHITE, Wendy, I., Germantown, MD 20876 (US)
(74) Representative: Walcher, Armin
(86) International application number: PCT/US2001/028877
(87) International publication number: WO 2002/024961

(56) References cited:
- CHRISTENSEN NEIL D ET AL: "Hybrid papillomavirus L1 molecules assemble into virus-like particles that reconstitute conformational epitopes and induce neutralizing antibodies to distinct HPV types" VIROLOGY, vol. 291, no. 2, 20 December 2001 (2001-12-20), pages 324-334, XP002280750 ISSN: 0042-6822
- ROSE R C ET AL: "Oral vaccination of mice with human papillomavirus virus-like particles induces systemic virus-neutralizing antibodies" VACCINE, BUTTERWORTH SCIENTIFIC. GUILDFORD, GB, vol. 17, no. 17, 23 April 1999 (1999-04-23), pages 2129-2135, XP004164997 ISSN: 0264-410X
- RODEN RICHARD B S ET AL: "Characterization of a human papillomavirus type 16 variant-dependent neutralizing epitope" JOURNAL OF VIROLOGY, vol. 71, no. 8, 1997, pages 6247-6252, XP002280751 ISSN: 0022-538X
- SCHILLER J.T. ET AL.: 'Papillomavirus-like particles and HPV vaccine development' CANCER BIOLOGY vol. 7, 1996, pages 373 - 382, XP000930048
- WHITE W.I. ET AL.: 'Characterization of a major neutralizing epitope on human papillomavirus type 16 L1' JOURNAL OF VIROLOGY vol. 73, no. 6, June 1999, pages 4882 - 4889, XP002907015

## Description

### Cross Reference to Related Application

This application claims priority from U.S. Provisional Application Serial No. 60/233,439, filed September 18, 2000, the entirety of which is incorporated herein by reference.

### Background of the Invention

### Field of the Invention

The present invention relates to the field of in vitro measurement of the immunogenicity of vaccines. In particular, the invention relates to the measurement of vaccine immunogenicity based on the binding properties of immunogenic and nonimmunogenic forms of vaccines based on Virus Like Particles.

### Summary of the Related Art

One of the critical factors in determining the stability of the bulk and vialed vaccine products is measuring immunogenicity. For example, potency testing of HPV-16 Virus-Like Particles (VLPs) by the immunization of mice is a labor and time-intensive endeavor. Typically, after immunization of the formulated VLP material into mice at various dose levels, it requires about six weeks before it can be determined whether the animals are able to mount a sufficient immune response. This problem becomes even more difficult when testing a variety of adjuvants and excipients because of the number of animals which must be utilized. In an effort to streamline development efforts it is desirable to find a rapid and reliable assay that allows the assessment of the immunogenicity of Virus Like Particle (VLP) based vaccines.

Ghim, SJ. et al. "Antigenicity of bovine papillomavirus type 1 (BPV-1) L1 virus-like particles compared that of intact BPV-1 virions" J. Gen. Virol., 77: 183 - 188, 1996 compares the antigenicity of intact bovine PV type 1 (BPV-1) virions with that of BPV-1 recombinant L1 VLP by ELISA using a well characterized panel of polyclonal and monoclonal antibodies generated against intact and denatured BPV-1 particles.

White, W.I. et al. "Characterization of a major neutralizing epitope on human papillomavirus type 16 L1" J. Virol., 73 (6): 4882 - 4889, 1999 describes the binding of three monoclonal antibodies to human papillomavirus type 16 L1 virus-like particles composed of reference L1 sequence and three variant L1 sequences.

### Summary and Objects of the Invention

It is an object of the invention to provide an assay for measuring the immunogenicity of a vaccine, wherein the vaccine comprises an epitope having a conformation associated with an immunogenically active form of the vaccine and a fragment having a conformation associated with an immunogenically inactive form of the vaccine. The method comprises exposing a sample of the vaccine to a first monoclonal antibody capable of binding to the epitope in the conformation associated with the immunogenically active form of the vaccine and a second monoclonal antibody capable of binding to the fragment in the conformation associated with the immunogenically inactive form of the vaccine and measuring the amount of first monoclonal antibody bound to the vaccine sample and the amount of the second monoclonal antibody bound to the vaccine sample. Preferably, the first and second monoclonal antibodies are each labeled with first and second detectable substances, respectively, prior to exposure of the vaccine thereto, and wherein the amount of first monoclonal antibody bound to the immunogenically active form of the vaccine and second monoclonal antibody bound to the immunogenically inactive form of the vaccine are determined by measuring the amount of first and second detectable substances associated with the vaccine sample upon exposure to the first and second monoclonal antibodies. Most preferably, the first and second detectable substances emit fluorescent light of different wave lengths when bound to the immunogenically active form of the vaccine and the immunogenically inactive form of the vaccine, respectively, and wherein measuring the amount of first and second monoclonal antibody bound to the vaccine is conducted by measuring the fluorescent light emitted by the first and second detectable substances. Typically, the vaccine sample comprises vaccine material and an adjuvant.

In a preferred embodiment, the vaccine sample is placed in a filterplate prior to exposure to the first and second monoclonal antibodies. The filterplate comprises two or more wells, wherein the vaccine sample is placed in at least one well and control adjuvant devoid of vaccine material is placed in at least another well not containing the vaccine material, and wherein the first and second labeled monoclonal antibodies are added to the wells in the plate. The wells containing the vaccine sample are washed upon addition of the first and second monoclonal antibodies to remove from the wells containing the vaccine sample excess monoclonal antibody that is not bound to the vaccine material prior to measuring the light emitted by the fluorescent substances.

The subject invention is particularly suitable for implementation of immunogenicity assays directed papillomavirus vaccine, particularly papillomavirus vaccine based on Virus Like particles comprising L1 protein, more particularly HPV-16 and HPV-18.

### Brief Description of the Drawings

Figure 1 shows data from the J4 filterplate assay showing the effect of thimerosal treatment on the J4, R5, and 18A1 monoclonal antibody binding patterns. The error bars represent the standard error of triplicates tested in the assay.
Figure 2 shows J4 assay data after 10 days of thimerosal treatment at 37°C showing the dramatic loss of J4 antibody binding in thimerosal treated samples. The error bars represent the standard error of triplicates run in the assay.
Figure 3 shows results from the J4 filterplate assay testing MEDI-517 (lot L98H074) that failed immunogenicity testing at the accelerated stability timepoint of 8 months. Because no control material was retained from the same lot, an additional lot of material at the same HPV-18 concentration was prepared and used as the control. The error bars represent the standard error of triplicates run in the J4 assay.
Figure 4 shows the effect of the thiol-specific organomercurial p-chloromercuriphenylsulfonic acid (PCMPS) on the J4 epitope of HPV-18 material as tested in the J4 filterplate assay. The error bars represent the standard error of triplicates run in the J4 assay.
Figure 5 shows the effect of overnight thimerosal treatment on the J4 epitope at the elevated temperature of 40°C. Loss of the J4 epitope was much accelerated over the 37°C storage condition, which required nearly 10 days to accomplish the same level of loss. The error bars represent the standard error of triplicates run in the J4 assay.

### Detailed Description of the Preferred Embodiments

The present invention is based on the discovery that the combination of conformational and linear epitope Mab binding can help to determine the relative state of denaturation of a vaccine sample. The invention is further based on the discovery of the correlation of conformational epitope binding with immunogenicity and neutralization. Based on these discoveries, it is believed that the correlative data obtained in conjunction with the development of the subject invention will allow an alternative route to immunogenicity of vaccines that eliminate or drastically reduce or the need for animal testing for immunogenicity associated with conventional methods of vaccine development and quality control.

The inventive concept of the subject application is particularly suitable for implementation using monoclonal antibodies that specifically bind an epitope or other fragment of vaccine material is various conformational states that are directly correlated to vaccine activity or inactivity.

While the subject application can be practiced in the testing and development of any vaccine that includes one or more fragments that may have conformations specifically associated with the active or the inactive state and being capable of binding with a traceable substance, the subject application will be described below by referring to Virus Like Particle based papillomavirus vaccines, specifically HPV-16 and HPV-18. However, the concepts illustrated through these vaccines can be easily generalized to a variety of other vaccines based on the foregoing specification.

The following materials are hereby incorporated by reference in their entirety:

Roden, R.B.S., Armstrong, A., Haderer, P., Christensen, N.D., Hubbert, N.L., Lowy, D.R., Schiller, J.T., and Kirnbauer, R. "Characterization of a Human Papillomavirus Type 16 Variant-dependent Neutralizing Epitope." J. Virol., 71:6247-52, 1997.

### EXAMPLE A: Immunoassay B for Measuring the Immunogenicity of HPV-16 Based Vaccine

This Example relates to an antigenicity assay that measures the binding of two fluorescently labeled monoclonal antibodies to probe HPV-16 VLP material on aluminum phosphate or aluminum hydroxide adjuvants. The V5 monoclonal antibody is directed towards the V5 structural epitope on the HPV-16 (VLP) surface which has been shown to be required for immunogenicity (3), while the 18A1 monoclonal antibody recognizes a linear epitope primarily present on degraded or denatured HPV-16 VLPs. This SOP applies to the general assessment of antigenicity on HPV-16 truncated VLP material adsorbed onto aluminum phosphate or aluminum hydroxide particles.

Utilizing the information from the experiments carried out by White, et al. that demonstrated the absolute necessity of the V5 structural epitope on the VLP surface for immunogenicity, we were able to construct an assay that correlated with in vivo immunogenicity in mice. By overexpressing the major HPV-16 capsid protein (L1) in a variety of expression systems it has been shown that, under the correct biochemical conditions, these subunits can assemble into virus-like particles (VLPs), often mimicking the native virus both structurally and immunologically. A number of monoclonal antibodies have been identified by Roden, et al., and two of these antibodies (HPV16.E70 and HPV16.V5) possess viral neutralizing activity against authentic HPV-16 virus. As reported by White, et al., mutation of three amino acids from the wild type L1 sequence, phenylalanine to leucine, alanine to threonine and lysine to asparagine at positions 50, 266 and 380, respectively, resulted in VLPs that were unable to be recognized by either the E70 or V5 monoclonal antibodies. Additionally, mice immunized with VLPs consisting of these mutations were found to be poorly immunogenic, and did not result in significant titers of neutralizing antibody. Mutation of a single V5 epitope amino acid at position 50 from leucine back to the wild type residue of phenylalanine resulted in not only the restoration of V5 antibody binding to the VLP, but also in the ability of the VLP to elicit antibody responses similar to the wild type L1 sequence in vivo.

The assay utilizes fluorescently labeled V5 antibody which binds to the immunogenic epitope on HPV-16 VLPs either formulated (adsorbed) onto aluminum particles, or unformulated bulk VLPs. There are two assay formats to accommodate each type of preparation. When VLPs were formulated on aluminum phosphate particles, the V5 filterplate format assay was the method of choice. This assay uses a 96-well filterplate to which small volumes of the formulated or control adjuvant materials (no VLPs) are added. The filter plate is mounted on a manifold, and a gentle vacuum is applied to deposit the aluminum phosphate particles on the well bottom to accomplish washing and separation steps. After blocking the particles for 1 hour with bovine serum albumin (BSA) the particles were washed, and fluorescently labeled V5 or 18A1 monoclonal antibodies were added (18A1 antibody preferentially recognizes a non-structural epitope on the HPV-16 VLP). The plate was then sealed and placed on a shaker for 2 hours at 37° C. The shaking step was included in order to keep the aluminum particles in suspension, thus keeping incubation times to a minimum. After a brief wash step the final pellet of material was resuspended with phosphate buffered saline (PBS) using a multi-channel pipette, and the plate was read on a fluorescent microplate reader with excitation at 485 nm and emission at 530 nm. The resulting V5 and 18A1 signals were background corrected, and the data was expressed as either a corrected signal, or as a percent of the control condition signal. Prior to the introduction of the filterplate assay, a microfuge tube-based assay using microcentrifugation to wash and separate phases was used. This was replaced by the filterplate version, which allowed greater sample throughput.

The V5 filterplate assay (DV-6363, ed. 001) uses an epitope-specific monoclonal antibody (V5) to determine the presence of the V5 structural epitope on aluminum phosphate formulated HPV-16 VLPs. Additionally, material is probed with a linear epitope recognizing monoclonal antibody (18A1), which provides additional information on the structural state of the formulated VLP. This assay uses a 96-well filterplate to which 50 µl volumes of the formulated or control adjuvant materials (no VLPs) are added. The filter plate is mounted on a manifold, and a gentle vacuum is applied to deposit the aluminum phosphate particles on the well bottom. After blocking the particles for 1 hour with a non-specific protein (bovine serum albumin) the particles were washed with PBS, and fluorescent-labeled V5 or 18A1 monoclonal antibodies were added to the wells. The plate was sealed and placed on a plate shaker for 2 hours at 37° C. The shaking step was included in order to keep the aluminum particles in suspension, thus keeping the incubation time to a minimum. After a brief wash step the final pellet of material was resuspended with phosphate buffered saline (PBS) using a 12-channel pipette, and the plate was read on a fluorescent microplate reader at 485_{EX}/530_{EM}. The resulting V5 and 18A1 signals were background corrected, and the data was expressed as either a corrected signal, or as a percent of the control condition signal. Prior to the introduction of the filterplate assay, early V5 assay data was generated using a microfuge tube-based assay using microcentrifugation to wash and separate the aluminum phosphate particles. This was replaced by the filterplate version, which allowed greater sample numbers to be efficiently run by one analyst.

### Preparation of Monoclonal Antibodies in Hollow-Fiber Reactors

Cells maintained in T-75 flasks (V5) were harvested by centrifugation, and resuspended in fresh media specific for that cell type. Frozen cell stocks (18A1) were thawed in a 37°C water, diluted with 20 ml of warm media, and the cells were harvested by centrifugation at 1,000 x g for 10 minutes. The supernatant was discarded, and the cell pellet was gently resuspended in 10 ml of fresh warm media and cultured overnight in a 25 ml tissue culture flask at 37°C. Cell flasks were examined daily, and fed or sub-cultured accordingly.

Following the hollow-fiber manufacturer's directions, approximately 3-5 x 10⁷ cells were harvested by centrifugation, resuspended in a 16 ml volume of fresh media, and inoculated into a hollow-fiber cartridge that had been previously conditioned with media according to the manufacturer's instructions. The hollow-fiber system was initially supplied with 125 ml of media in the reservoir. Cell growth was monitored by measuring the amount of lactate produced, and the 125 ml reservoir was replaced with fresh medium in 500 ml and 1 L reservoir sizes when lactate levels in the reservoir medium exceeded 1 gram per liter. Generally, the cartridge could be harvested once the 1L media reservoir was required, which typically was 2 weeks post-inoculation of the cartridge. The lactate levels were monitored daily, and harvesting was performed upon each media change. The harvest material was cleared of cells and debris by centrifugation at 2,000 x g for 10 minutes, and the resulting supernatant was removed and frozen at -20°C until purification could be performed. A small aliquot from each harvest was analyzed by ELISA to determine the level of mAb produced prior to purification. Typically, each hollow-fiber reactor was harvested every 2^{nd} day, and could be harvested for over 8 weeks with no loss in productivity.

Our experience with the filterplate assay has been gathered over a very short period of time, since the assay was only in existence for several months. However, during that time a number of performance characteristics and trends were determined during the method's optimization and sample testing. Assay optimization data is limited since the assay performed very well from its inception. Optimization efforts centered on three areas, namely, sample volume reduction, reduction in the amount of labeled antibody required per reaction well, and evaluation of different blocking reagents.

The first set of experiments varied the amount of formulated VLP material and amount of labeled antibody added per reaction well in the 96-well filterplate. HPV-16 material formulated with SBAS4 was tested at concentrations of 5 or 2 g/ml (125 or 50 l/well, respectively) with labeled V5 or 18A1 at concentrations of 10, 5, or 2.5 g/well. The assay was performed as described in SOP DV-6363 with the exception that the assay diluent also contained 0.1% (v/v) of non-specific mouse IgG. The results from this experiment are summarized in Tables 1 and 2. The data from Table 1 shows that based upon the signal/noise ratio the optimal 18A1 or V5 antibody concentration to use with 5 µg/well of adjuvanted material is 2.5 µg/well. Table 2 shows data generated at the same antibody concentrations with 2 µg/ml of adjuvanted material, and yields similar results. Based on these experiments the volume of adjuvanted material to test per well was set at 50 µl (2 µg/well) with labeled V5 or 18A1 antibody levels being set at 2.5 µg/well. The Alexa-488 labeling levels of the V5 and 18A1 antibodies used in this experiment were 4.3 and 1.1, respectively.

The original V5 tube method used a non-specific mouse IgG₂ₐ, in addition to BSA, as a blocking agent. The addition of this secondary blocking agent was found to yield superior background in the tube assay format over that of BSA alone. This blocking procedure was investigated again when the assay format was changed to the 96-well filterplate format because of the drastic reduction in the amount of AlPO₄ and antibody material used per assay sample. Tables 3 and 4 show the results of the blocking study, and compares the use of blocking agent containing BSA alone, or BSA with 10 µg/ml of non-specific mouse IgG₂ₐ used as the block and diluent in the filterplate assay. This study was performed with HPV-16 formulated antigen with a 50 µl (2 µg/well) sample volume, and labeled antibody amounts of 2.5 µg/well, previously determined to be optimal for the system. The Alexa-488 labeling levels of the V5 and 18A1 antibodies used in this study were 4.3 and 1.1, respectively. As seen in Tables 3 and 4, the removal of the mouse IgG₂ₐ actually improved the assay performance by reducing the %CV of the replicates from each group, as well as by increasing the signal/noise ratio observed. Therefore, based on these studies it was decided to omit the non-specific mouse IgG₂ₐ from the filterplate-based V5 assay.

The most critical performance characteristic observed in this assay system was the labeling levels of the V5 and 18A1 with the Alexa-488 fluorescent dye. Initially, we labeled 1-mg amounts of V5 or 18A1 and obtained varying labeling levels, often less than the 4-mole minimum mentioned in the kit instructions. Because of this, and the manufacturer's claim that Alexa-488 was relatively insensitive to self-quenching, we purchased bulk amounts of Alexa-488-SE (succinimidyl ester) so that we could label one large bulk amount of V5 or 18A1 antibody. Our bulk labeling effort, described above, followed essentially the same procedure as listed in DV-5204 with the exception that the 12.5 mg of V5 or 18A1 antibody was labeled in one bulk amount with the same proportions of antibody and Alexa-488-SE that had been custom-packaged by the vendor. This bulk labeling approach resulted in labeling levels of 6.7 and 9.4 moles of incorporation per mole of 18A1 and V5, respectively.

Upon using these bulk labeled reagents in the assay at identical concentrations to the old batch labeled reagents, it was discovered that AlPO₄-adsorbed HPV-16 VLPs exhibited much less signal than was observed when the old reagents with lower labeling efficiencies were used. In an additional study, VLPs that were adsorbed on AlPO₄ at 40 or 4 µg/ml (and tested at 2 and 0.2 µg/well) were stained with the bulk or batch-labeled V5 and 18A1 preparations. Our results demonstrated that the V5 and 18A1 at lower labeling levels had near the expected ten-fold drop in signal between the 40 and 4 µg/ml materials, while the highly labeled bulk V5 and 18A1 had only 2-3.5 fold signal modulation. Moreover, the V5 signal for the lower labeling-level batch V5 was about 7-fold higher than the bulk-labeled V5 when used to probe the 40 µg/ml AlPO₄-adsorbed samples.

A similar phenomenon was observed for the bulk and batch labeled 18A1 antibodies. This data is shown in Table 5. From our limited experiments it cannot be conclusively determined whether the signal reduction observed for the bulk-labeled materials was due to fluorophore quenching or loss of antibody binding, but the limited data we gathered suggests the former. If loss of binding was responsible for the signal loss, one would expect to see a similar drop in the batch:bulk signal ratio. For example, the six-fold signal difference for the 40 µg/ml material probed with V5 should be observed for the 4 µg/ml material as well. This constant ratio was not observed, which suggests that fluorescence quenching is occurring when higher levels of VLP material are probed. The hypothesis is that at higher VLP adsorption levels, the heavily labeled V5 antibody is in a more sterically constrained state, allowing the Alexa-488 molecules to be in close proximity to one another. This steric organization results in Alexa-488 self-quenching, in a manner identical to that reported for fluorescein.

Although the instructions supplied with the Alexa-488 labeling kits (specifically designed to label IgG) stated an acceptable coupling range of 4-9 moles of Alexa-488 per mole of IgG, our experience has been that any levels in excess of 5.4 moles of label result in a significant loss of signal. This was based on the batch labeling experiment that labeled 2 mg of IgG per reaction vial, which is twice the level of protein recommended by the supplier of the labeling kit. This resulted in a 5.4 molar labeling ratio, and also a satisfactory signal ratio for the 2 VLP doses tested (see Table 5). Based upon our findings we have changed the range of incorporation from the vendor recommended 4-9 moles of label per mole of antibody to 1-5.4 moles of incorporation. DV-5204 will be updated accordingly.

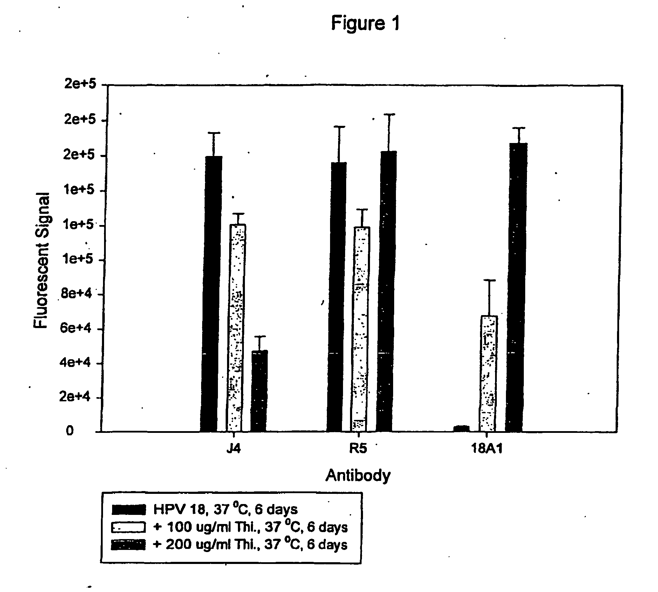

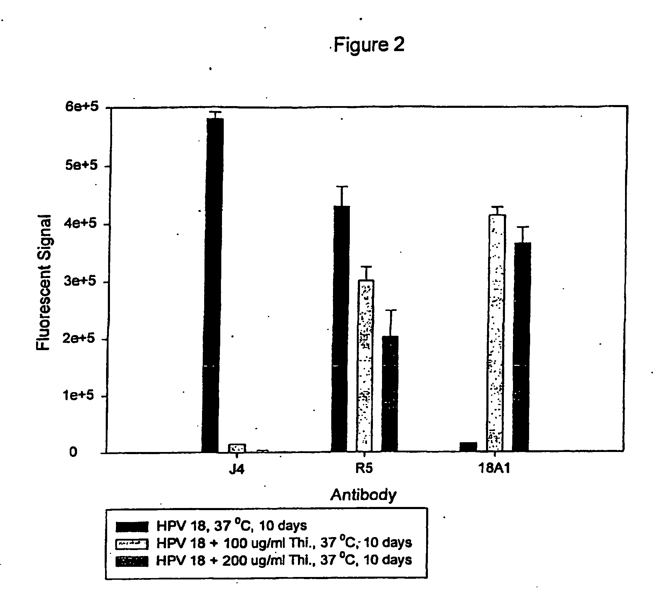

Additional data generated using the V5 filterplate method is shown in Table 6. Figures 1 and 2 show the V5 and 18A1 signals generated when comparing two lots of HPV-16 materials formulated on AlPO₄ (SBAS4) with and without thimerosal. The data shows that when compared with non-thimerosal containing material, formulated material with thimerosal underwent almost a complete loss of V5 binding ability despite being stored at 4°C (Fig. 1). Also, the loss in V5 binding was corroborated by the large increase in 18A1 binding signal observed (Fig. 2), which is indicative of breakdown of the VLP. This breakdown exposes additional linear epitopes to which the 18A1 monoclonal preferentially binds.

Table 6 provides data on a study to determine if unlabeled antibody (V5) can be used in the filterplate assay and subsequently detected with a commercially available Alexa-488 labeled anti-mouse conjugate (Molecular Probes). In this study formulated material (MEDI-503.1 w/SBAS4 at 40 µg/ml) was treated with 1 mM p-chloromercuriphenylsulfonic acid (PCMPS) at RT for 30 minutes to intentionally degrade the V5 epitope. The filterplate assay was run using directly labeled V5 and 18A1 (standard method), as well as with varying concentrations of unlabeled V5 subsequently detected with the anti-mouse Alexa-488 conjugate. As shown in Table 6, there was no apparent correlation between the two methods, indicating that using unlabeled antibody was not feasible for the V5 filterplate assay.

In summary, this example provides a detailed of the V5 filterplate assay and its use in evaluating the antigenicity of VLP based vaccines.

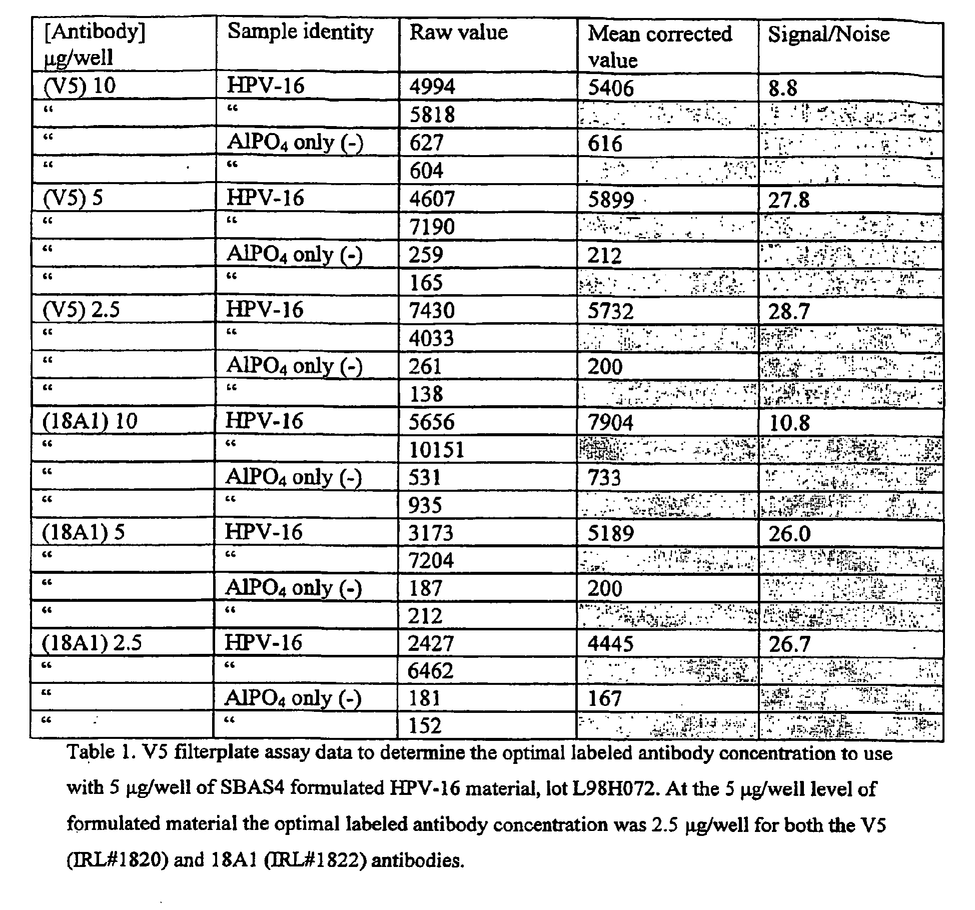

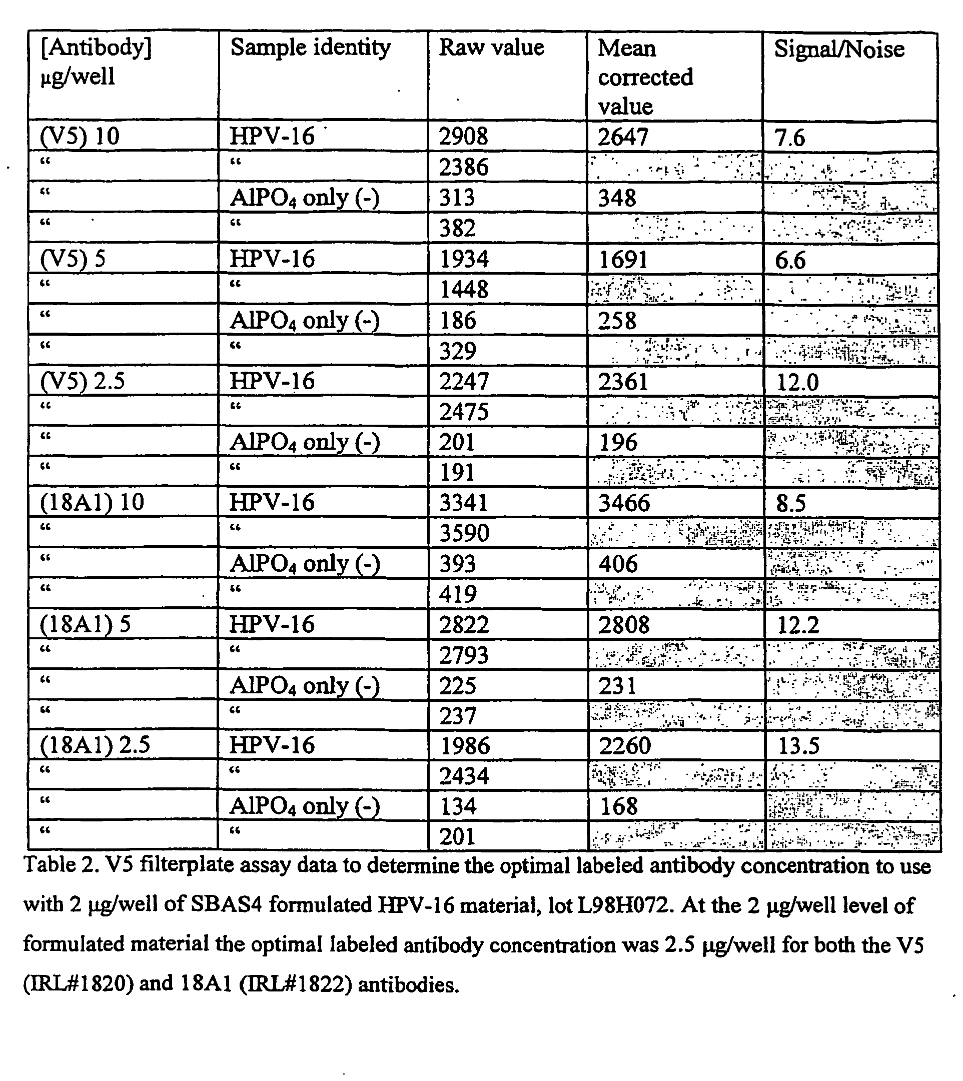

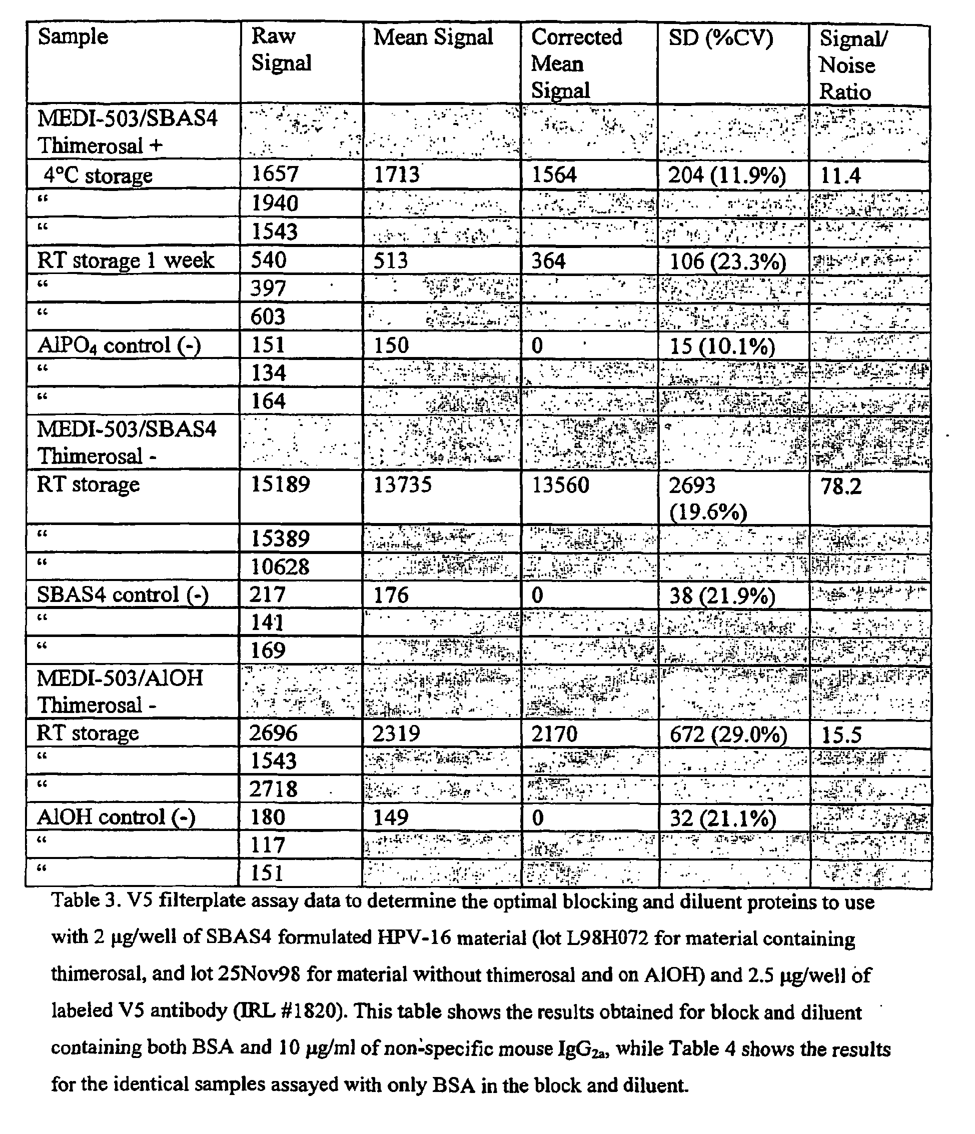

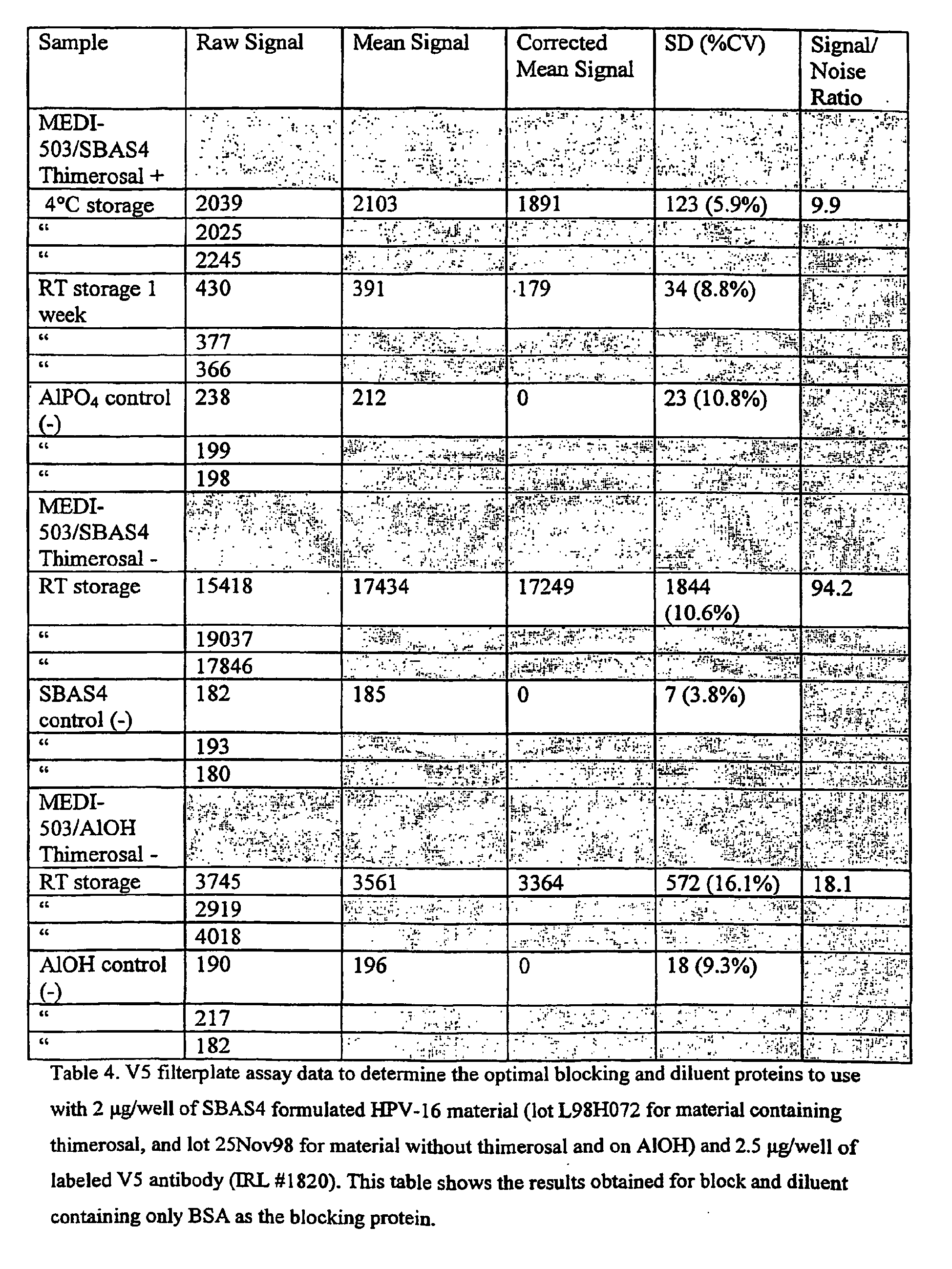

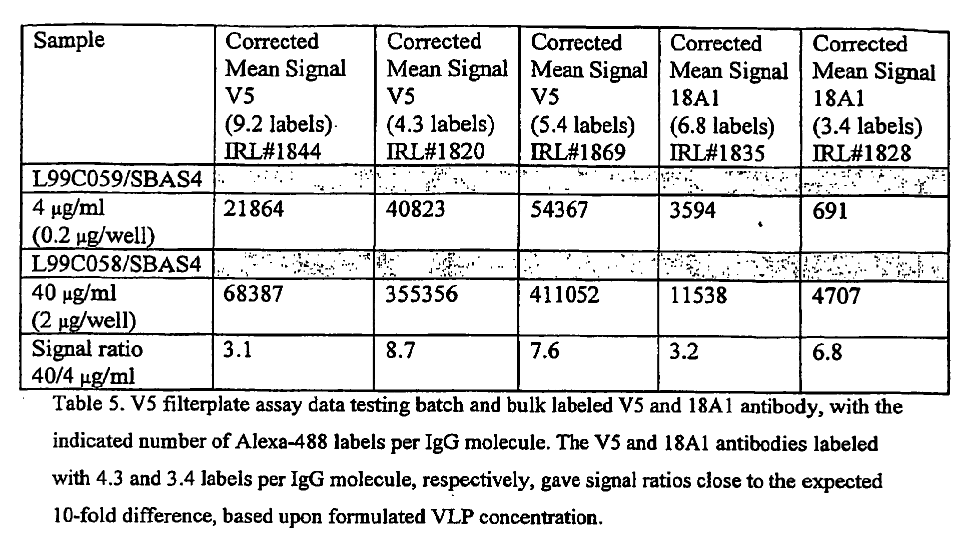

**Table 6. Comparison of results obtained from directly labeled V5 antibody (standard filterplate method) with the variation of using unlabeled V5 detected with a secondary Alexa-488 labeled conjugate.**

| Antibody | Sample | Mean corrected signal | % of control signal |
|---|---|---|---|
| Alexa-488 labeled V5 (standard method) | formulated HPV-16, untreated | 399576 | 100 |
| Alexa-488 labeled V5 (standard method) | Formulated HPV-16, 1 mM PCMPS (30 min. RT) | 86295 | 22 |
| 10 µg/ml V5 unlabeled (test method) | formulated HPV-16, untreated | 118531 | 100 |
| 10 µg/ml V5 unlabeled (test method) | Formulated HPV-16, 1 mM PCMPS (30 min. RT) | 107670 | 91 |
| 20 µg/ml V5 unlabeled (test method) | formulated HPV-16, untreated | 104163 | 100 |
| 20 µg/ml V5 unlabeled (test method) | Formulated HPV-16, 1 mM PCMPS (30 min. RT) | 101317 | 97 |
| 25 µg/ml V5 unlabeled (test method) | formulated HPV-16, untreated | 122826 | 100 |
| 25 µg/ml V5 unlabeled (test method) | Formulated HPV-16, 1 mM PCMPS (30 min. RT) | 94742 | 77 |

### EXAMPLE B: Immunoassay B for Measuring the Immunogenicity of HPV-18 Based Vaccine

This Example relates to the J4 filterplate assay, which is an antigenicity assay that measures the presence of the J4 epitope on HPV-18 aluminum-adsorbed mono-bulks (AMB), and on formulated materials. The J4 monoclonal antibody is directed towards the J4 structural epitope on the HPV-18 VLP which is required for immunogenicity, while the 18A1 monoclonal antibody recognizes a linear epitope exposed on degraded material. The VLP material can be adsorbed to either aluminum phosphate or aluminum hydroxide particles.

In an effort to develop an assay for HPV-18 similar to the assay developed for HPV-16 as described in Example A , experiments were performed in which AlOH-adsorbed material was treated with thimerosal and stained with several structure specific and a linear epitope specific monoclonals. This was done to screen for antibodies which potentially may be of use in an assay similar to the V5 assay, but for the HPV-18 VLP. This approach suggested that of the four structure-specific monoclonals tested, only the J4 epitope was affected by thimerosal treatment. The structural monoclonal R5 showed no change in binding as a result of thimerosal treatment. The linear epitope specific monoclonal (18A1) also showed dramatic changes in response to thimerosal treatment. The optimized HPV-18 antigenicity assay utilizes both the J4 and 18A1 monoclonal antibodies. Subsequent immunogenicity studies that were run in parallel with the J4 assay supported the hypothesis that the J4 epitope was required for immunogenicity.

The J4 filterplate assay uses epitope-specific monoclonal antibody (J4) to determine the presence of the structural epitope on aluminum hydroxide and aluminum phosphate formulated HPV-18 VLPs. Additionally, material is probed with a linear epitope recognizing monoclonal antibody (18A1), which provides additional information on the structural state of the formulated VLP. This assay uses a 96-well filterplate to which 2 g of the formulated or control adjuvant materials (no VLPs) are added. The filter plate is mounted on a manifold, and a gentle vacuum is applied to deposit the aluminum particles on the well bottom. After blocking the particles for 1 hour with a non-specific protein (bovine serum albumin), the particles are washed with phosphate buffered saline (PBS), and the J4 or 18A1 monoclonal antibodies are added to the wells. The plate is sealed and placed on a plate shaker for one hour at 37° C. The shaking step is used to keep the aluminum particles in suspension, thus keeping the incubation time to a minimum. A wash step is performed after the incubation, and fluorescent-labeled rabbit anti-mouse IgG is added to the wells. After the incubation and wash steps, the final pellet of material is resuspended with PBS using a 12-channel pipette, and the plate is read on a fluorescent microplate reader at 485_{EX}/530_{EM}. The resulting J4 and 18A1 signals were background corrected, and the data was expressed as either a corrected signal, or as a percent of the control condition signal.

### Purification of J4 Monoclonal Antibodies Using Protein A/G Column

The Protein A/G column and buffers were allowed to warm to room temperature prior to use. The frozen J4 ascites was thawed, diluted with an equal volume of ImmunoPure IgG Binding buffer, and applied to the A/G column previously equilibrated with 10 ml of Binding Buffer. After the sample entered the resin, the column was washed with 20 ml of Binding Buffer. The bound IgG was eluted with 10 ml of ImmunoPure IgG Elution Buffer, and collected into 0.1 fraction volumes of 1 M Tris, pH 7.5. The column was regenerated using 8 ml of Elution buffer followed with several column volumes of PBS containing 0.02% sodium azide, and stored at 4 °C. The purified antibody was concentrated using a CentriPrep-30 device with centrifugation for 30 min. at 3,000 rpm. The antibody was then dialyzed in 4 liters of PBS containing 0.02% sodium azide overnight at 4 °C. The concentration of the antibody was determined using A₂₈₀ absorbance with an extinction coefficient of 1.4.

### Preparation of 18A1 and R5 Monoclonal Antibodies in Hollow-Fiber Reactors

A frozen cell stock was rapidly thawed in a 37°C water bath, diluted with 20 ml of pre-warmed media, and the cells were harvested by centrifugation at 1,000 x g for 10 minutes. The supernatant was discarded, and the cell pellet was gently resuspended in 10 ml of fresh media and cultured overnight in a 25 ml tissue culture flask at 37°C. Cell flasks were examined daily, and fed or sub-cultured accordingly.

Approximately 3-5 x 10⁷ cells were harvested by centrifugation, resuspended in a 16 ml volume of fresh media, and inoculated into a preconditioned hollow-fiber cartridge that had been previously conditioned with 100 ml of media in a 37 °C humidified incubator for at least 48 hours. The hollow-fiber system was initially supplied with 125 ml of media in the reservoir. Cell growth was monitored by measuring the amount of lactate produced, and the 125 ml reservoir was replaced with fresh medium in 500 ml and 1 liter reservoir sizes, respectively, when lactate levels in the reservoir medium exceeded 1 gram per liter. Generally, the cartridge could be harvested once the 1 liter media reservoir was required, which typically was 2 weeks post-inoculation of the cartridge. The lactate levels were monitored daily, and harvesting was performed upon each media change. The harvest material was cleared of cells and debris by centrifugation at 2,000 x g for 10 minutes, and the resulting supernatant was removed and frozen at -20°C until purification could be performed. A small aliquot from each harvest was analyzed using a direct ELISA (antigen down) to determine the level of monoclonal produced prior to purification. Typically, each hollow-fiber reactor could be harvested every other day, and the culture could be maintained for a minimum of 8 weeks with no loss in productivity.

### Purification of 18A1 and R5 Monoclonal Antibody Using Macro-Prep Ceramic Hydroxyapatite Chromatography

Monoclonal antibody was purified from the hollow-fiber harvests using ceramic hydroxyapatite chromatography. Twenty grams of Macro-Prep Ceramic Hydroxyapatite resin was rehydrated, and a column was poured following the procedure, "Packing Small Columns Using Slurry Packing," supplied by the manufacturer. The packed column was equilibrated with 5 bed volumes (∼100 ml) of 10 mM PO₄ buffer, pH 6.8, at a flow rate of 7-10 ml/min. One tube (∼ 30 ml) of frozen harvest material was thawed; 0.2 m filtered, and diluted 1:4 with deionized water. The diluted harvest was loaded onto the column, and the column was washed with equilibration buffer until the 280 nm UV absorbance of the effluent returned to a stable baseline. The bound monoclonal antibody was eluted using a NaCl gradient from 0-1 M (Buffer A = 10 mM PO₄, pH 6.8; Buffer B = 10 mM PO₄ + 1 M NaCl, pH 7.2), with a typical elution occurring between 300-400 mM NaCl for 18A1. The purified antibody was concentrated to a final protein concentration of 1-2 mg/ml, and dialyzed against several changes of Dulbecco's Phosphate Buffered Saline (D-PBS), without calcium or magnesium, using 10,000 Dalton cutoff dialysis tubing. The purity of the material was determined by capillary gel electrophoresis (CGE), and has consistently had purity greater than 90%. The column was regenerated by rinsing with 100 ml of 400 mM PO₄, pH 6.8, followed by 120 ml of 1 M NaOH at a reduced flow rate of 2 ml/min. The column was then rinsed with 400 mM PO₄, pH 6.8, at a 10 ml/min flow rate until the effluent pH measured 6.8. The column was finally rinsed with 100 ml of 10 mM PO₄, pH 6.8, containing 0.02% sodium azide, and stored upright at RT.

The J4 assay adopted the same sample and antibody concentrations used in the V5 filterplate assay. Assay optimization was minimal because the assay performed well using these conditions. The major focus of this method optimization was to determine if any of the structure-specific monoclonals available to us were predictive of immunogenicity. Previously, we have shown that the V5 epitope, required for HPV-16 immunogenicity, was degraded by treatment of the material with the organomercurial thimerosal. Further experimentation showed that the thimerosal treated HPV-16 material no longer elicited an antibody response in immunized mice. Our goal was to use thimerosal as a tool to see if any of the structure-specific monoclonals that we had access to showed a loss of binding to thimerosal treated HPV-18 VLPs, and if that loss of binding had any relation to immunogenicity in mice.

The HPV-18 material formulated on aluminum phosphate was treated with either 100 or 200 µg/ml of thimerosal at 37°C for either 6 or 10 days. This material, along with the untreated control, were analyzed in the J4 assay using the J4 and R5 structure-specific antibodies, as well as the 18A1 linear-epitope specific antibody. The J4 binding signal decreased after treatment of the material with thimerosal for 6 days, and was completely gone after 10 days (Figures 1 and 2). The R5 signal was unaffected after 6 days of thimerosal treatment, and only slightly decreased after 10 days of treatment. Similar to the HPV-16 reaction, the 18A1 binding increased after the thimerosal treatment. This result suggested that the J4 epitope was sensitive to organomercurial degradation, and warranted that immunogenicity studies be started.

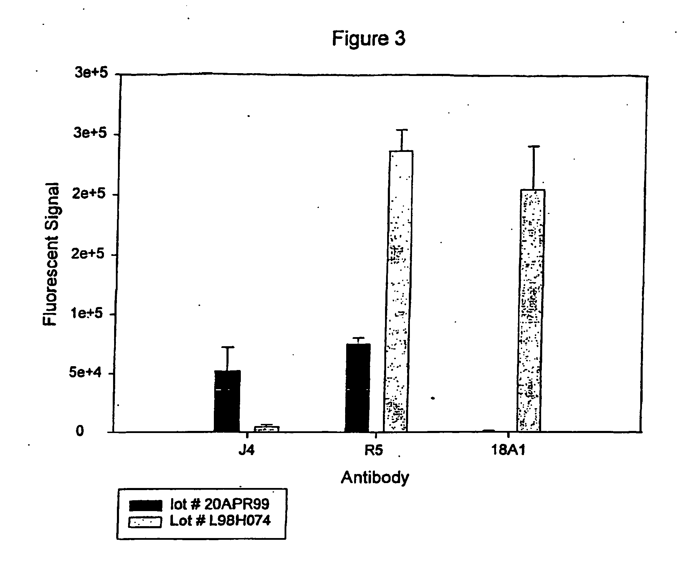

The first set of data supporting the hypothesis that the J4 epitope was required for immunogenicity in mice was elicited after testing formulated HPV-18 material (lot #L98H074) that had failed the immunogenicity test after undergoing accelerated stability testing (SCW 971:59). This material had a low-level of J4 signal compared to control material at an equivalent concentration (lot #20Apr99). The R5 binding signal was unaffected, and the 18A1 binding signal was elevated indicating degradation (see Figure 3). This data supports the hypothesis that the J4 epitope is involved in immunogenicity.

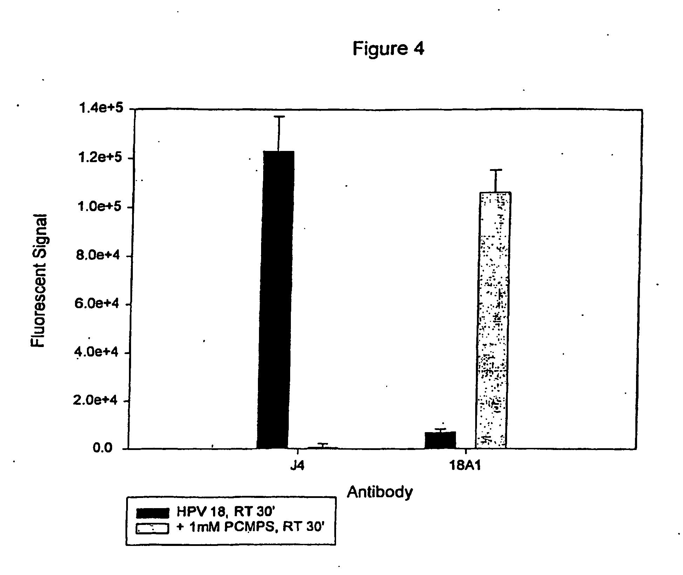

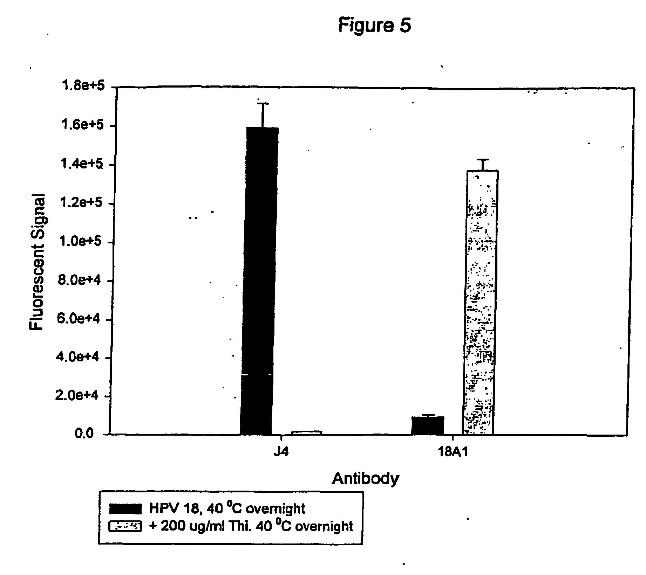

To verify that the epitope bound by the J4 monoclonal was also required for immunogenicity, it was necessary to perform the J4 assay in parallel with mouse immunizations using thimerosal treated and untreated material. Because thimerosal degradation requires several days even at elevated temperatures, the organomercurial PCMPS (p-chloromercuriphenylsulfonic acid) was tested for its effect on the J4 epitope. This compound was a much better characterized substance than thimerosal with documented effects on thiol groups. HPV-18 VLPs were treated with either 200 µg/ml thimerosal at 40°C overnight, or with 1 mM PCMPS at room temperature for 30 minutes. All of the samples were analyzed in the HPV-18 antigenicity (J4) filterplate assay. The thimerosal treated material lost J4 signal and had increased 18A1 signal when compared to the untreated control (see Figure 4). The PCMPS treatment showed decreased J4 signal and increased 18A1 signal (see Figure 5), which was similar to the effect observed with thimerosal treatment. This data showed that PCMPS had the same effect on the J4 epitope as thimerosal, but with accelerated kinetics.

The role of the J4 epitope in immunogenicity was examined in a study in which formulated HPV-18 material was treated with 200 µg/ml thimerosal, and tested in the J4 and immunogenicity assay in parallel. A shown in Table 1, at the 200 ng dose the seroconversion rate was only 40% in the thimerosal-treated material at sera dilutions of 1:200, 400, or 800, but was 100% in the untreated control at these same dilutions. The J4 signal of the thimerosal treated immunogen was 49% when normalized to the control (untreated) signal. Table 2 shows the result of an additional antigenicity/immunogenicity study using PCMPS. In this experiment HPV-18 material (MEDI-504.2, MJA 02Jul99) was treated with 1 mM PCMPS at room temperature for 30 minutes, and then put on ice to stop the degradation. These samples were analyzed using the J4 antigenicity assay, and also immunized into mice at doses of 2 and 0.2 µg/mouse. As shown in Table 2, the PCMPS treated material that had only a 2% J4 signal (normalized to untreated control) elicited no seroconversion at any sera dilution at the 0.2 µg dose. A large loss of potency was also observed at the 2 µg dose. The R5 signal was only slightly reduced, suggesting that some structural integrity of the molecule was left intact. The studies summarized in Tables 1 and 2 strongly suggest that the J4 epitope is required for HPV-18 VLP immunogenicity (potency) in mice.

Therefore, this assay provides an in vitro means of evaluating the potential efficacy of a VLP based vaccine based on antigenicity.

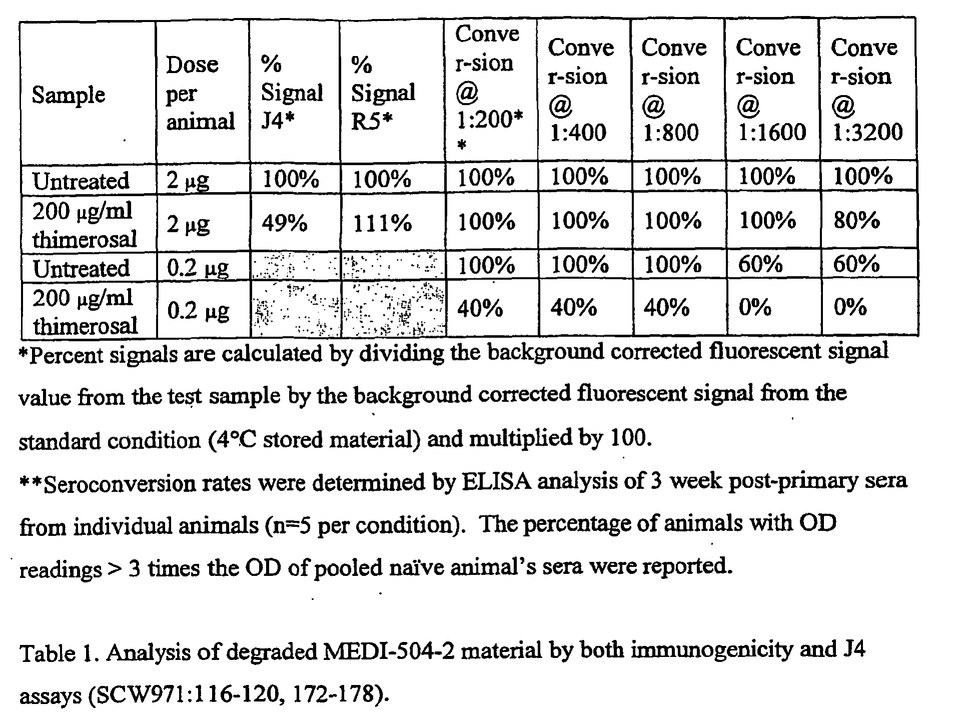

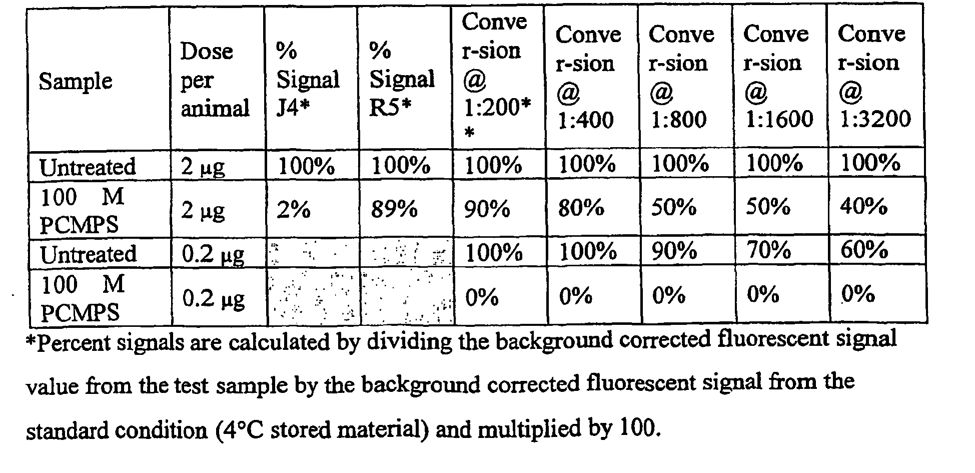

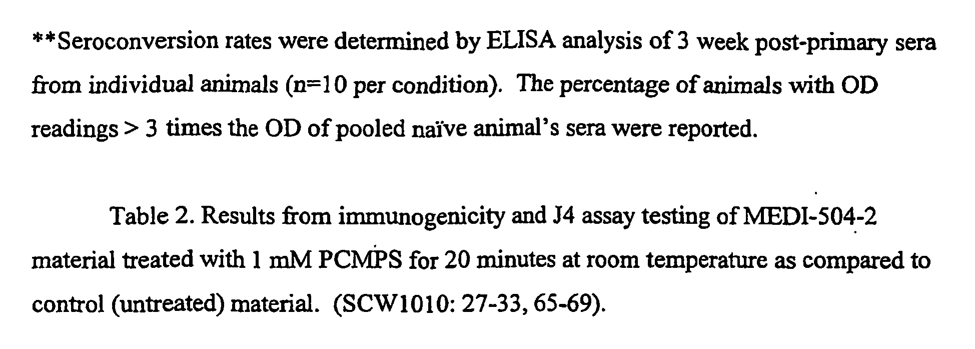

## Claims

1. An assay for measuring the immunogenicity of a vaccine, wherein the vaccine comprises an epitope having a conformation associated with an immunogenically active form of the vaccine and a fragment having a conformation associated with an immunogenically inactive form of the vaccine, wherein the method comprises exposing a sample of the vaccine to a first monoclonal antibody capable of binding to the epitope in the conformation associated with the immunogenically active form of the vaccine and a second monoclonal antibody capable of binding to the fragment in the conformation associated with the immunogenically inactive form of the vaccine and measuring the amount of first monoclonal antibody bound to the vaccine sample and the amount of the second monoclonal antibody bound to the vaccine sample.

2. The assay of Claim 1, wherein the first and second monoclonal antibodies are each labeled with first and second detectable substances, respectively, prior to exposure of the vaccine thereto, and wherein the amount of first monoclonal antibody bound to the immunogenically active form of the vaccine and second monoclonal antibody bound to the immunogenically inactive form of the vaccine are determined by measuring the amount of first and second detectable substances associated with the vaccine sample upon exposure to the first and second ligands.

3. The assay of Claim 2, wherein the first and second detectable substances emit fluorescent light of different wave lengths when bound to the immunogenically active form of the vaccine and the immunogenically inactive form of the vaccine, respectively, and wherein measuring the amount of first and second monoclonal antibody bound to the vaccine is conducted by measuring the fluorescent light emitted by the first and second detectable substances.

4. The assay of Claim 3, wherein the vaccine sample comprises vaccine material and an adjuvant

5. The assay of Claim 4, wherein the vaccine sample is placed in a filterplate prior to exposure to the first and second monoclonal antibodies.

6. The assay of Claim 5, wherein the filterplate comprises two or more wells, wherein the vaccine sample is placed in at least one well and control adjuvant devoid of vaccine material is placed in at least another well not containing the vaccine material, and wherein the first and second labeled monoclonal antibodies are added to the wells in the plate.

7. The assay of Claim 6, wherein the wells containing the vaccine sample are washed upon addition of the first and second monoclonal antibodies to remove from the wells containing the vaccine sample excess monoclonal antibody that is not bound to the vaccine material prior to measuring the light emitted by the fluorescent substances.

8. The assay of Claim 1, wherein the vaccine is a papillomavirus vaccine.

9. The assay of Claim 8, wherein the vaccine comprises a Virus Like Particle.

10. The assay of Claim 9, wherein the Virus Like Particle comprises L1 protein.

11. The assay of Claim 9, wherein the Virus Like Particle comprises HPV-16.

12. The assay of Claim 11, wherein the epitope having a conformation associated with an active form of the vaccine is V5 epitope

13. The assay of Claim 12, wherein the monoclonal antibody capable of binding to the epitope in a conformation associated with the immunogenically active form of the vaccine is HPV16.V5 monoclonal antibody.

14. The assay of Claim 13, wherein HPV 16.V5 is labeled with a fluorescent substance.

15. The assay of Claim 11, wherein the inactive form of the vaccine comprises denatured L1 protein.

16. The assay of Claim 15, wherein the denatured L1 protein comprises linear L1 protein.

17. The assay of Claim 9, wherein the Virus Like Particle comprises HPV-18.

## Patentansprüche

1. Test zur Messung der Immunogenizität eines Impfstoffes, wobei der Impfstoff ein Epitop mit einer Konformation im Zusammenhang mit einer immunogen aktiven Form des Impfstoffes und ein Fragment mit einer Konformation im Zusammenhang mit einer immunogen inaktiven Form des Impfstoffes umfasst, wobei das Verfahren die Exposition einer Probe des Impfstoffes mit einem ersten monoklonalen Antikörper, der zur Bindung an das Epitop in der Konformation im Zusammenhang mit der immunogen aktiven Form des Impfstoffes befähigt ist, und mit einem zweiten monoklonalen Antikörper, der zur Bindung an das Fragment in der Konformation im Zusammenhang mit der immunogen inaktiven Form des Impfstoffes befähigt ist, und die Messung der Menge des ersten monoklonalen Antikörpers, der an die Impfstoffprobe gebunden ist, und der Menge des zweiten monoklonalen Antikörpers, der an die Impfstoffprobe gebunden ist, umfasst.

2. Test nach Anspruch 1, wobei der erste und zweite monoklonale Antikörper jeweils mit ersten bzw. mit zweiten nachweisbaren Substanzen markiert worden ist, bevor die Exposition des Impfstoffes damit erfolgt, und wobei die Menge des ersten monoklonalen Antikörpers, der an die immunogen aktive Form des Impfstoffes gebunden ist, und die Menge des zweiten monoklonalen Antikörpers, der an die immunogen inaktive Form des Impfstoffes gebunden ist, bestimmt werden, indem man die Mengen der ersten und zweiten nachweisbaren Substanz, die mit der Impfstoffprobe bei Exposition mit dem ersten und zweiten monoklonalen Antikörper assoziiert ist, misst.

3. Test nach Anspruch 2, wobei die erste und zweite nachweisbare Substanz fluoreszierendes Licht mit unterschiedlichen Wellenlängen emittieren, wenn sie an die immunogen aktive Form des Impfstoffes bzw. an die immunogen inaktive Form des Impfstoffes gebunden sind, und wobei die Messung der Mengen des ersten und zweiten monoklonalen Antikörpers, die an den Impfstoff gebunden sind, durch Messung des fluoreszierenden Lichts, das von der ersten und zweiten nachweisbaren Substanz emittiert wird, durchgeführt wird.

4. Test nach Anspruch 3, wobei die Impfstoffprobe Impfstoffmaterial und ein Adjuvans umfasst.

5. Test nach Anspruch 4, wobei die Impfstoffprobe vor der Exposition mit dem ersten und zweiten monoklonalen Antikörper auf einer Filterplatte platziert wird.

6. Test nach Anspruch 5, wobei die Filterplatte zwei oder mehr Vertiefungen umfasst, wobei die Impfstoffprobe in mindestens eine Vertiefung platziert wird und Kontrolladjuvans, das frei von Impfstoffmaterial ist, in mindestens eine weitere Vertiefung, die kein Impfstoffmaterial enthält, platziert wird und wobei der erste und zweite markierte monoklonale Antikörper den Vertiefungen in der Platte zugesetzt werden.

7. Test nach Anspruch 6, wobei die Vertiefungen, die die Impfstoffprobe enthalten, nach Zugabe des ersten und zweiten monoklonalen Antikörpers gewaschen werden, um aus den Vertiefungen, die die Impfstoffprobe enthalten, überschüssigen monoklonalen Antikörper, der nicht an das Impfstoffmaterial gebunden ist, vor der Messung des durch die fluoreszierenden Substanzen emittierten Lichts zu entfernen.

8. Test nach Anspruch 1, wobei es sich beim Impfstoff um Papillomavirus-Impfstoff handelt.

9. Test nach Anspruch 8, wobei der Impfstoff ein virusartiges Teilchen umfasst.

10. Test nach Anspruch 9, wobei das virusartige Teilchen L1-Protein umfasst.

11. Test nach Anspruch 9, wobei das virusartige Teilchen HPV-16 umfasst.

12. Test nach Anspruch 11, wobei es sich beim Epitop mit einer Konformation in Zusammenhang mit einer aktiven Form des Impfstoffes um ein V5-Epitop handelt.

13. Test nach Anspruch 12, wobei es sich bei dem monoklonalen Antikörper, der zur Bindung an das Epitop in einer Konformation im Zusammenhang mit der immunogen aktiven Form des Impfstoffes befähigt ist, um monoklonalen HPV-16.V5-Antikörper handelt.

14. Test nach Anspruch 13, wobei HPV-16.V5 mit einer fluoreszierenden Substanz markiert ist.

15. Test nach Anspruch 11, wobei die inaktive Form des Impfstoffes denaturiertes L1-Protein umfasst.

16. Test nach Anspruch 15, wobei das denaturierte L1-Protein lineares L1-Protein umfasst.

17. Test nach Anspruch 9, wobei das virusartige Teilchen HPV-18 umfasst.

## Revendications

1. Dosage destiné à mesurer l'antigénicité d'un vaccin, dans lequel le vaccin comprend un épitope ayant une conformation associée à une forme antigéniquement active du vaccin et un fragment ayant une conformation associée à une forme antigéniquement inactive du vaccin, dans lequel le procédé comprend l'exposition d'un échantillon du vaccin à un premier anticorps monoclonal capable de se lier à l'épitope dans la conformation associée à la forme antigéniquement active du vaccin et un deuxième anticorps monoclonal capable de se lier au fragment dans la conformation associée à la forme antigéniquement inactive du vaccin et la mesure de la quantité du premier anticorps monoclonal lié à l'échantillon de vaccin et de la quantité du deuxième anticorps monoclonal lié à l'échantillon de vaccin.

2. Dosage selon la revendication 1, dans lequel le premier et le deuxième anticorps monoclonal sont chacun marqués par une première et une deuxième substance détectable, respectivement, avant l'exposition du vaccin à ceux-ci, et dans lequel la quantité du premier anticorps monoclonal lié à la forme antigéniquement active du vaccin et du deuxième anticorps monoclonal lié à la forme antigéniquement inactive du vaccin est déterminée en mesurant la quantité des première et deuxième substances détectables associées à l'échantillon de vaccin après exposition au premier et au deuxième ligand.

3. Dosage selon la revendication 2, dans lequel la première et la deuxième substance détectable émettent une lumière fluorescente de différentes longueurs d'onde lorsqu'elles sont liées à la forme antigéniquement active du vaccin et à la forme antigéniquement inactive du vaccin, respectivement, et dans lequel la mesure de la quantité du premier et du deuxième anticorps monoclonal lié au vaccin est réalisée en mesurant la lumière fluorescente émise par la première et la deuxième substance détectable.

4. Dosage selon la revendication 3, dans lequel l'échantillon de vaccin comprend le vaccin et un adjuvant.

5. Dosage selon la revendication 4, dans lequel l'échantillon de vaccin est placé sur une plaque filtrante avant l'exposition au premier et au deuxième anticorps monoclonal.

6. Dosage selon la revendication 5, dans lequel la plaque filtrante comprend deux puits ou plus, dans lequel l'échantillon de vaccin est placé dans au moins un puits et un adjuvant témoin dépourvu de vaccin est placé dans au moins un autre puits ne contenant pas le vaccin, et dans lequel le premier et le deuxième anticorps monoclonal sont ajoutés aux puits de la plaque.

7. Dosage selon la revendication 6, dans lequel les puits contenant l'échantillon de vaccin sont lavés après addition du premier et du deuxième anticorps monoclonal pour éliminer des puits contenant l'échantillon de vaccin, l'anticorps monoclonal en excès qui n'est pas lié au vaccin avant de mesurer la lumière émise par les substances fluorescentes.

8. Dosage selon la revendication 1, dans lequel le vaccin est un vaccin de papillomavirus.

9. Dosage selon la revendication 8, dans lequel le vaccin comprend une pseudo particule virale.

10. Dosage selon la revendication 9, dans lequel la pseudo particule virale comprend une protéine L1.

11. Dosage selon la revendication 9, dans lequel la pseudo particule virale comprend HPV-16.

12. Dosage selon la revendication 11, dans lequel l'épitope ayant une conformation associée à une forme active du vaccin est l'épitope V5.

13. Dosage selon la revendication 12, dans lequel l'anticorps monoclonal capable de se lier à l'épitope dans une conformation associée à la forme antigéniquement active du vaccin est l'anticorps monoclonal HPV16.V5.

14. Dosage selon la revendication 13, dans lequel HPV16.V5 est marqué par une substance fluorescente.

15. Dosage selon la revendication 11, dans lequel la forme inactive du vaccin comprend la protéine L1 dénaturée.

16. Dosage selon la revendication 15, dans lequel la protéine L1 dénaturée comprend une protéine L1 linéaire.

17. Dosage selon la revendication 9, dans lequel la pseudo particule virale comprend HPV-18.
